(19) Europäisches Patentamt
European Patent Office
Office européen des brevets

(11) **EP 2 940 467 B1**

(12) **FASCICULE DE BREVET EUROPEEN**

(45) Date de publication et mention de la délivrance du brevet:
**20.02.2019 Bulletin 2019/08**

(51) Int Cl.:
***G01N 33/18*** *(2006.01)*

(21) Numéro de dépôt: **15164847.4**

(22) Date de dépôt: **23.04.2015**

(54) **Méthode de détermination de la reprotoxicité d'eaux douces**

Verfahren zur Bestimmung der Reproduktionstoxizität von Frischwasser

Determination method for the reproductive toxicity of fresh water

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.04.2014 FR 1453885**

(43) Date de publication de la demande:
**04.11.2015 Bulletin 2015/45**

(73) Titulaire: **Institut National de Recherche en Sciences et Technologies pour l'Environnement et l'Agriculture (IRSTEA)
92761 Antony Cedex (FR)**

(72) Inventeurs:
• **Chaumot, Arnaud**
 **69001 LYON (FR)**
• **Geffard, Olivier**
 **17220 ST VIVIEN (FR)**

(74) Mandataire: **Schmidt, Martin Peter
IXAS Conseil
15, rue Emile Zola
69002 Lyon (FR)**

(56) Documents cités:
• OLIVIER GEFFARD ET AL: "Ovarian cycle and embryonic development in Gammarus fossarum: Application for reproductive toxicity assessment", ENVIRONMENTAL TOXICOLOGY AND CHEMISTRY, vol. 29, no. 10, 20 août 2010 (2010-08-20) , pages 2249-2259, XP055137849, ISSN: 0730-7268, DOI: 10.1002/etc.268
• ROMAIN COULAUD ET AL: "feeding assay with(Crustacea): Modelling the influence of confounding factors to improve water quality biomonitoring", WATER RESEARCH, ELSEVIER, AMSTERDAM, NL, vol. 45, no. 19, 15 septembre 2011 (2011-09-15), pages 6417-6429, XP028103989, ISSN: 0043-1354, DOI: 10.1016/J.WATRES.2011.09.035 [extrait le 2011-09-22]
• Tim A Verslycke ET AL: "Critical Review MYSID CRUSTACEANS AS POTENTIAL TEST ORGANISMS FOR THE EVALUATION OF ENVIRONMENTAL ENDOCRINE DISRUPTION: A REVIEW", Environmental Toxicology and Chemistry, 1 janvier 2004 (2004-01-01), pages 1219-1234, XP055138357, Extrait de l'Internet: URL:http://onlinelibrary.wiley.com/store/1 0.1897/03-332/asset/5620230516_ftp.pdf?v=1 &t=hzo8yeop&s=7dfcce7c54cf24c622f678ceb43 1 38cc811269e7 [extrait le 2014-09-04]

## Description

### Domaine technique

L'invention concerne le domaine des méthodes d'analyses écotoxicologiques des eaux. Plus particulièrement, elle concerne la détermination de la reprotoxicité des eaux douces à l'aide d'une sonde biologique, c'est-à-dire d'un organisme aquatique qui sert comme modèle pour caractériser l'état de reprotoxicité d'une eau douce dans laquelle il est introduit pour y séjourner pendant une durée déterminée aux fins de l'essai.

### Etat de la technique

L'utilisation d'organismes aquatiques comme indicateur écotoxicologique pour le diagnostic et la surveillance de l'état écotoxicologique d'une eau douce est connue en tant que telle. Parmi les organismes aquatiques, les crustacés ont été identifiés comme un taxon adapté à cette utilisation : ils sont ubiquistes dans les eaux douces de l'Europe, facile à prélever et à élever, faciles à calibrer et à garder en cage dans un flux d'eau à surveiller.

Plusieurs documents se focalisent sur les daphnies : WO 2013/081496 décrit un dispositif de test impliquant cet organisme, RU 2491546 décrit une méthode pour estimer la génotoxicité à partir de l'observation de la seconde génération de daphnies, RU 2377560 décrit une méthode d'exposition de daphnies à une eau naturelle ou un effluent permettant de déterminer aisément leur mortalité, DE 4110877 utilise l'analyse de leurs mouvements tels que détectés par une cellule photoélectrique, WO 90112886 utilise l'analyse par fluorescence d'une activité enzymatique des daphnies.

Une autre famille de crustacés étudiée dans ce contexte est les gammaridés. Les demandes de brevet DE 19848230 (Almut Gerhardt), DE4313109 (Liess) et SU2029948 (Université de Saint Petersbourg) décrivent la détection de changements comportementaux de gammaridés induits par le degré de pollution de l'eau dans laquelle ils séjournent. Plus particulièrement, le premier document décrit la modification du mouvement des animaux et l'analyse spectrale des variations de champ magnétique induites par leurs mouvements, le second décrit la détection de la remontée à la surface des animaux en fonction du degré de pollution, alors que le troisième présente une autre analyse de leurs mouvements. Ces méthodes sont plus fines que celles qui comptabilisent simplement la mortalité des animaux, et elles permettent de caractériser plus spécifiquement la neurotoxicité des eaux.

D'autres méthodes impliquant des crustacés détectent une réponse métabolique spécifique au stress qu'ils subissent dans leur milieu aquatique, telle que la production de certains « protéines de choc » (WO 90/13028 et WO 90/02947).

Encore d'autres méthodes utilisent des sondes biologiques comme des algues (EP 2 177 905), l'étude comportementale de poissons ou de mouches (WO 2008/150096), l'évaluation de la fertilité d'oeufs de mollusques (EP 1 256 800) ou du degré d'ouverture des coquilles d'une moule (DE 4443788), un poisson transgénique particulier (US 2004/147030), des embryons de mouches (US 6,365,129), des cultures de rotifères (US 5,798,222), des embryons et larves d'échinidés (RU 2 057 337).

Plusieurs publications décrivent l'utilisation du crustacé *Gammarus fossarum* comme biosonde pour l'évaluation de la toxicité d'eaux douces naturelles.

Ainsi, la publication « Acetylcholinesterase activity in Gammarus fossarum (Crustacea Amphpoda) - Intrinsic variability, reference levels, and a reliable tool for field surveys » par B. Xuereb et al., parue dans la revue Aquatic Toxicology 93 (2009), p. 225-233, décrit l'utilisation d'une population de mâles *Gammarus fossarum* de taille standardisée comme sonde pour caractériser l'effet de polluants neurotoxiques à travers l'activité du biomarqueur acétylcholine-estérase.

La publication « Linking genotoxic responses in Gammarus fossarum germ cells with reproduction impairement, using the Comet assay" par E. Lacaze et al., parue dans la revue Environmental Research 111 (2011), p. 626-634, décrit une corrélation entre l'endommagement de l'ADN induit par des substances génotoxiques dans le sperme de *Gammarus fossarum* et l'apparition d'anomalies embryonnaires.

La publication « Vitellogenin-like proteins in the freshwater amphipod Gammarus fossarum (Koch, 1835): Functional characterization throughout reproductive process, potential or use as an indicator of oocyte quality and endocrine disruption biomarkers in males" par G. Jubeaux et al., parue dans la revue Aquatic Toxicology 112-113 (2012), p. 72-82, décrit l'influence de certains perturbateurs endocriniens sur la concentration de la protéine vitellogénine, qui caractérisent le cycle de reproduction de *Gammarus fossarum.*

La publication "Ovarian cycle and embryonic development in Gammarus fossarum: Application for reproductive toxicity assessment" par O. Geffard et al., parue dans la revue Environmental Toxicology and Chemistry 29 (10), p. 2249-2259 (2010), décrit l'influence de contaminants sur le cycle de développement gonadique et embryonnaire chez Gammarus *fossarum.*

La publication "In situ feeding assay with Gammarus fossarum (Crustacea): Modelling the influence of confounding factors to improve water quality biomonitoring" par R. Coulaud et al., parue dans la revue Water Research 45 (2011), p. 6417-6429, décrit la détermination d'un indice d'inhibition d'alimentation chez *Gammarus fossarum,* qui caractérise l'effet toxique de contaminants dans l'environnement.

Ainsi, les macro-invertébrés aquatiques apparaissent comme des candidats prometteurs pour développer une biosonde pour la caractérisation écotoxicologique d'eaux douces. Cependant, il manque une méthode pour caractériser la reprotoxicité d'eaux douces à l'aide d'une biosonde représentative, ubiquiste dans les eaux douces d'Europe, facile à prélever, à élever et à manipuler, facile à calibrer et à garder en cage dans un flux d'eau à surveiller, et facile à observer et à analyser ; proposer une telle méthode représente le problème que l'invention cherche à résoudre.

**Objets de l'invention**

L'invention est basée sur la découverte que la synchronisation entre l'embryogénèse, la vitellogenèse et les stades de mue chez les *Gammaridae,* et en particulier chez le genre *Gammarus,* peut être perturbée par des substances toxiques et/ou par des substances perturbant le système hormonal de cette espèce d'invertébré (perturbateurs endocriniens) , et qu'il est possible de distinguer entre ces deux phénomènes perturbateurs ; ainsi il est possible d'utiliser les *Gammaridae,* et en particulier les espèces du genre *Gammaridae,* comme biosonde pour caractériser la toxicité, et en particulier la reprotoxicité, d'une eau douce. Cette caractérisation de l'eau naturelle se fait par une exposition des organismes de la famille *Gammaridae* à l'eau à caractériser, et elle peut se faire en site naturel, malgré les variations de température qu'une telle eau naturelle peut subir au cours de la durée de l'exposition. On sait en effet que de nombreux phénomènes physiologiques et cycles biologiques chez les *Gammaridae*, et en particulier chez *Gammarus*, dépendent de la température de l'eau ; tel est le cas du cycle de mue. Les présents inventeurs ont résolu cette difficulté par un procédé nouveau qui permet de tenir compte des effets de température non seulement lors de l'interprétation des données collectées lors d'un tel essai, mais encore lors de la conception de l'essai même. En effet, le procédé selon l'invention implique de définir en fonction de la température du milieu naturel certains paramètres d'exposition sur lesquels l'expérimentateur peut agir pour tenir compte de cette température, et en particulier la durée de certaines étapes d'exposition des *Gammaridae* à leur milieu naturel à caractériser.

Plus précisément, selon l'invention le problème est résolu par un procédé pour déterminer l'état de reprotoxicité d'une eau douce à l'aide de crustacés d'une espèce sélectionnée parmi les crustacés de la famille des *Gammaridae,* et de préférence sélectionné parmi les crustacés du genre *Gammarus,* dans lequel procédé

(A) on approvisionne des crustacés d'une même espèce composé de mâles et de femelles en état de précopulation, à savoir un premier lot M de mâles et un deuxième lot de crustacés adultes en couple C en état de précopulat, ledit lot C étant composé de mâles et de femelles, lesdites femelles étant sélectionnées de manière à être toutes prêtes à pondre et à muer dans le jour ou les deux jours qui suivent ;

(B) on met les lots M et C dans l'eau douce à caractériser pendant une première période d'exposition de durée DE1 prédéterminée, et on détermine la température moyenne T1 de l'eau au cours de ladite première période d'exposition DE1 ;

(C) à l'issue de la durée d'exposition DE1 on (C.a) récupère le lot M, et on (C.b) continue à exposer ledit lot C pendant une deuxième période d'exposition de durée DE2 prédéterminée, et on détermine la température moyenne T2 de l'eau au cours de cette deuxième période d'exposition DE2, et on (C.c) détermine pour chacun des crustacés femelles du lot C : (i) le stade de développement embryonnaire (paramètre appelé ici « critère d'embryogénèse ») $E_c$, et (ii) un paramètre qui représente la vitellogénèse, et qui est de préférence la surface ovocytaire (paramètre appelé ici « critère de vitellogénèse ») $V_b$, et (iii) le stade de mue $M_a$ ; et on (C.d) évalue la synchronisation entre les critères du stade de mue, de vitellogénèse et d'embryogénèse, et si $M_a$, $V_b$ et $E_c$ sont synchronisés on évalue si la distribution statistique des femelles dans les différents stades de mue est conforme à une distribution de référence obtenue dans des conditions de non-toxicité.

La durée DE2 de ladite deuxième période d'exposition peut être déterminée à partir de la température moyenne T1 de l'eau au cours de ladite première durée d'exposition de durée DE1.

Dans un mode de réalisation, on détermine la consommation d'aliments solides des crustacés du lot M au cours de ladite durée DE1. On vérifie ainsi si le lot M montre une inhibition de l'alimentation. Cette détermination peut se faire avec une source d'alimentation solide en feuilles de surface prédéterminée.

Dans un mode de réalisation, on vérifie si $M_a$, $V_b$ et $E_c$ sont synchronisés et la distribution statistique des femelles dans les différents stades de mue est conforme à une distribution de référence obtenue dans des conditions de non-toxicité, et si ces conditions sont remplies on conclut que l'eau à caractériser ne présente pas de toxicité, alors que si $M_a$, $V_b$ et $E_c$ sont synchronisés et la distribution statistique des femelles dans les différents stades de mue n'est pas conforme à une distribution de référence obtenue dans des conditions de non-toxicité, on conclut que l'eau à caractériser présente une toxicité générale.

Si $M_a$, $V_b$ et $E_c$ ne sont pas synchronisés et on constate une inhibition de l'alimentation, on conclut que l'eau à caractériser présente une toxicité générale, et si $M_a$, $V_b$ et $E_c$ ne sont pas synchronisés et on constate l'absence d'inhibition de l'alimentation on conclut que l'eau à caractériser provoque une toxicité spécifique, de type perturbation endocrinienne,

La durée d'exposition DE2 est déterminée en déterminant le pourcentage attendu de femelles en stade de mue AB, C1, C2, D1 et D2 au bout de n jours lors d'une exposition à températures variables. Dans ce but on peut procéder de la manière suivante :

(a) on détermine la température moyenne $T_n$ de chaque jour *n*,

(b) pour chacune de ces *n* températures Tn on calcule une durée *d* en jours qui correspond au temps qui serait nécessaire pour le crustacé pour passer à cette température du début du cycle de mue initial au début du stade de mue D2,

(c) en utilisant ces *n* durées *d* on calcule le pourcentage *p* de réalisation des 4 premiers stades de mue à la fin des *n* jours,

(d) à partir du pourcentage *p* on calcule les probabilités *q* d'avoir réalisé la transition entre chacun des cinq stades à la fin des n jours,

(e) à partir de ces probabilités *q* on détermine les proportions de femelles attendues respectivement aux stades AB, C1, C2, D1 et D2 au bout de *n* jours.

Dans une première variante de ce mode réalisation on choisit la durée DE2 de manière à ce que le nombre de femelles au stade de mue C2 soit maximum. Dans une deuxième variante on choisit la durée DE2 de manière à ce qu'au moins 60%, de préférence au moins 70%, et encore plus préférentiellement au moins 80% des femelles soient au stade de mue C2.

Le procédé selon l'invention peut être mise en oeuvre de manière avantageuse avec des crustacés de l'espèce *Gammarus fossarum.* Dans ce cas, on approvisionne à l'étape A des crustacés de l'espèce *Gammarus fossarum* et à l'étape (C.c) :

(a) pour déterminer le stade de mue $M_a$ on considère que a = 1 pour le stade de mue AB qui regroupe les stades de post-mue A et B, a = 2 pour le stade de mue C1, a = 3 pour le stade de mue C2, a = 4 pour le stade de mue D1, a = 5 pour le stade de mue D2,

(b) pour déterminer le critère de la vitellogénèse $V_b$ on considère que : b = 1 si la surface ovocytaire est comprise entre 20 100 $\mu m^2$ et 23 700 $\mu m^2$, b = 2 si la surface ovocytaire est comprise entre 34 900 $\mu m^2$ et 43 900 $\mu m^2$, b = 3 si la surface ovocytaire est comprise entre 99 200 $\mu m^2$ et 112 800 $\mu m^2$, b = 4 si la surface ovocytaire est comprise entre 129 700 $\mu m^2$ et 136 300 $\mu m^2$, b = 5 si la surface ovocytaire est comprise entre 158 200 $\mu m^2$ et 169 800 $\mu m^2$;

(c) pour déterminer le critère de l'embryogénèse $E_c$ on considère que c = 1 au stade de l'embryon nouvellement fertilisé, ovale et indifférencié, c = 2 au stade de l'apparition de la ventroflexion, c = 3 au stade de l'apparition du céphalothorax et de la segmentation des appendices, c = 4 au stade où les yeux sont clairement visibles (ommatidies) et les appendices sont complètement développés, c = 5 au stade du juvénile nouvellement éclos.

Dans cette mise en oeuvre avantageuse avec l'espèce *Gammarus fossarum,* on considère que $M_a$, $V_b$ et $E_c$ sont synchronisés si a = b = c, et que $M_a$, $V_b$ et $E_c$ ne sont pas synchronisés si cette condition a = b = c n'est pas remplie.

Chez *Gammarus fossarum,* le stade de mue est déterminé à partir de l'analyse fine des évolutions tégumentale du dactylopodite et du protopodite de la première et deuxième paire de périopodes.

La méthode selon l'invention peut être utilisée pour caractériser des eaux douces naturelles.

D'une manière générale, les températures T1 et T2 doivent être compatibles avec la survie des crustacés choisis. Pour les *Gammaridae* elles sont avantageusement comprises entre 0°C et 28°C. Avantageusement, ces températures se situent dans la zone d'occurrence naturelle des espèces de *Gammaridae.*

## Figures

La figure 1 montre de manière schématique la corrélation entre la fertilité (exprimée en nombre d'ovocytes par femelle) et le stade de mue de femelles de *Gammarus fossarum.* La taille médiane est de 8,3 mm avec un interquartile de 1 mm (n=80 femelles).

La figure 2 montre de manière schématique la corrélation entre la consommation alimentaire et la taille corporelle de *Gammarus fossarum.*

La figure 3 montre de manière schématique la corrélation entre la consommation alimentaire de *Gammarus fossarum*. et la température de l'eau.

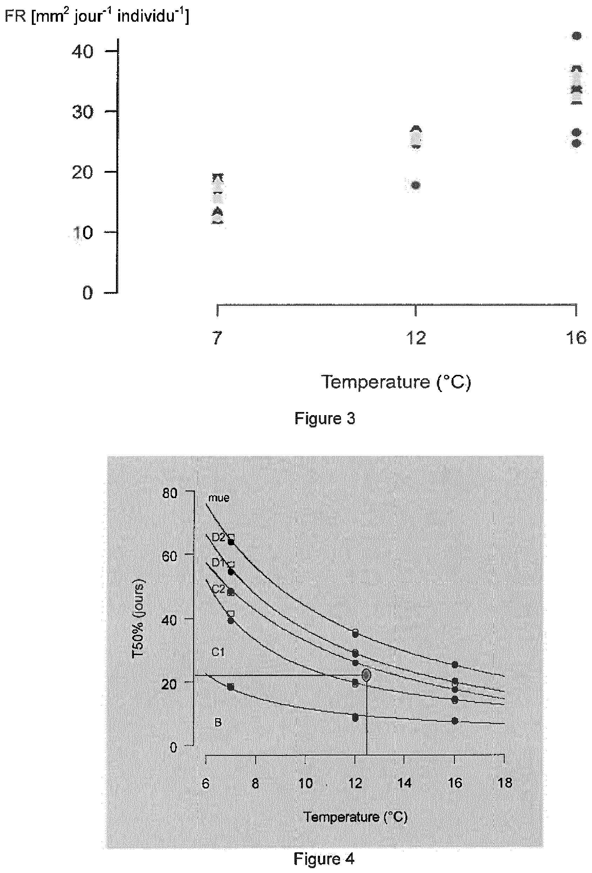

La figure 4 montre de manière schématique la relation entre la température et la durée des différents stades du cycle de mue chez *Gammarus fossarum*. Les carrés blancs correspondent aux observations pour la conductivité de 600 $\pm$ 50 $\mu$S cm$^{-1}$ et les points noirs pour la conductivité de 200 $\pm$ 50 $\mu$S cm$^{-1}$. Le point caractérisé par ses renvois aux axes correspond à une condition d'essai en laboratoire où l'on obtient en milieu contrôlé (21 jours à 12,5°C) une majorité de femelles au stade de mue C2. Les courbes ont été calculées par un modèle linéaire généralisé pour chacun des cinq stades de mue.

La figure 5 montre de manière schématique l'anatomie d'un représentant du genre *Gammarus*. On distingue le prosoma (P), le mesosoma (S), le metasola (T) et le urosoma (U), ainsi que : le céphalon (C), l'ocelle (O), le telson (E), les uropodes (U1), les pléopodes (P1), les péréiopodes (P2), les gnathopodes (G), l'antenne (A1), l'antennule (A2) avec la flagelle principale (F2) et la flagelle accessoire (F1).

## Description détaillée

Nous décrivons ici un procédé complet de détermination du degré de toxicité d'une eau douce à caractériser, qui inclut le procédé selon l'invention.

Etape (1) : On approvisionne des individus (crustacés) d'une même espèce de la famille des gammares, de préférence du genre *Gammarus,* et encore plus préférentiellement de l'espèce *Gammarus fossarum*.

Etape (2) : On garde les crustacés en quarantaine pendant une durée prédéterminée DQ (typiquement comprise entre 15 jours et quatre semaines), dans des conditions de température (de préférence 12°C), éclairage (cycle 16-8 ou, de préférence, cycle 14-10) et d'alimentation (*ad libitum*) contrôlées, dans une eau non-contaminée.

Etape (3) : A l'issue de la durée de quarantaine DQ, on sélectionne un premier lot M de crustacés mâles non parasités, les crustacés du premier lot ayant de préférence une taille homogène proche d'une taille de référence (pour les crustacés de l'espèce *Gammarus fossarum* entre 9.5 et 11.5 mm en moyenne), et on sélectionne un deuxième lot de crustacés adultes en couple C en état de précopulat, les femelles devant être synchronisées entre elles en termes d'état reproductif, celles-ci devant toutes être prêtes à pondre et à muer (mue prévue un ou deux jours après la date de tri).

Etape (4) : On met un nombre prédéterminé de crustacés du premier lot M et du deuxième lot C en cages dans l'eau à caractériser pendant une première durée d'exposition DE1 prédéterminée (typiquement 7 jours), et on détermine les températures moyennes journalières de l'eau au cours de ladite durée d'exposition DE1. On alimente les crustacés du lot M en début de test à partir d'une source d'alimentation solide et on détermine à l'issue de ladite durée DE1 la consommation d'alimentation solide.

Etape (5) : A l'issue de la durée d'exposition prédéterminée DE1 :

(a) On récupère le lot M de mâles,
(b) On détermine la durée d'exposition DE2 d'une deuxième période de prolongation de l'exposition pour le lot de couples C à l'aide d'une méthode P1 décrite ci-dessous.
(c) On continue à exposer ledit lot C pendant ladite deuxième période d'exposition de durée DE2 à l'eau à caractériser, et on détermine les températures moyennes journalières de l'eau sur ladite durée d'exposition DE2.
(d) On récupère le lot de couples C à l'issue de la période DE2, ainsi que les enregistrements de température pendant la période DE2. Le lot C est ramené au laboratoire.
(e) On détermine une durée de stabulation post-exposition DPE et la température de stabulation pour le lot C de couples, de préférence selon une méthode P2 qui est décrite ci-dessous.
(f) On effectue pour ledit lot C de couples une stabulation pendant la durée DPE et à la température T3.
(g) On détermine pour chacun des crustacés femelles du lot C (au moins 10, de préférence au moins 15 femelles) :

(i) le stade de développement embryonnaire (ce paramètre étant appelé ici « critère d'embryogénèse ») $E_c$, et
(ii) la surface ovocytaire (ce paramètre étant appelé ici « critère de vitellogénèse) $V_b$, et
(iii) le stade de mue $M_a$.

A ce stade, on dispose d'une part d'un enregistrement des températures (toutes les heures) au cours de l'expérimentation (depuis le tri initial, le transport sur site, l'exposition sur site, le retour au laboratoire et l'éventuelle stabulation de post-exposition) et d'autre part, de la détermination du stade de mue (AB, C1, C2, D1 ou D2) de chacune des femelles récupérées en fin de test. Pour obtenir des résultats statistiquement significatifs, on doit analyser au moins 10, et de préférence au moins 15 femelles.

Etape (6) : A partir des résultats obtenus à l'étape 5 (g) on évalue, de préférence en utilisant la méthode P3 décrite ci-dessous, la présence ou l'absence d'un retard ou d'un décalage anormal dans le déroulement du cycle de reproduction au cours de l'exposition *in situ*, un tel décalage étant indiqué par la désynchronisation entre le critère d'embryogénèse, le critère de vitellogénèse et le stade de mue. Plus précisément :

(a) Evaluation de la synchronisation entre les critères du stade de mue, de la vitéllogénèse et de l'embryogénèse. Le critère de toxicité procède d'une interprétation simultanée des trois critères de mue, de vitellogénèse et d'embryogénèse.
Si $M_a$, $V_b$ et $E_c$ sont synchronisés tel que a=b=c, alors on constate qu'il n'y a pas de désynchronisation. Dans le cas contraire on constate qu'il y a une désynchronisation.

(b) Dans le cas où il y a désynchronisation on continue la procédure avec l'étape 7.

(c) Dans le cas où il n'y a pas de désynchronisation on effectue une analyse quantitative des données pour déterminer s'il y a un retard anormal dans le déroulement du cycle de reproduction au cours de l'exposition *in situ* (i.e. au cours de l'exposition à l'eau à caractériser) :

(i) A partir de l'enregistrement journalier des températures (pendant la durée d'exposition DE1+DE2+DPE), on calcule grâce à la méthode P1 décrite ci-dessous les proportions attendues de femelles dans les différents stades. Ces proportions correspondent à la distribution de référence attendue en cas de non-toxicité au cours de l'exposition.

(ii) Les pourcentages de femelles dans les différents stades de mue sont alors comparés statistiquement entre observations et distribution de référence.

(iii) Si cette analyse montre un décalage entre les données observées et les données de référence, alors on décide que l'eau présente une toxicité générale. Dans le cas contraire on décide que l'eau ne présente pas de toxicité vis-à-vis du déroulement du cycle de mue et de reproduction.

Etape (7) : Dans le cas où à l'étape 6(b) on conclut à la présence d'une désynchronisation, on analyse les données qui représentent l'impact de l'exposition à l'eau à caractériser sur l'alimentation des mâles, telles qu'obtenues à l'étape 4.

(a) Si l'on constate un impact sur l'alimentation, selon l'écart de référence, on conclut que l'eau présente une toxicité générale.

(b) Si l'on constate l'absence d'impact sur l'alimentation on constate que l'eau à caractériser provoque une toxicité spécifique, de type perturbation endocrinienne.

Ainsi, le procédé aboutit à une conclusion parmi trois conclusions possibles par rapport à la qualité de l'eau caractérisée : « pas de toxicité générale », « toxicité générale », « toxicité spécifique, de type perturbation endocrinienne ».
Afin de ne pas alourdir la description du procédé selon l'invention, nous décrivons ici de manière séparée et de manière très détaillée quatre méthodes désignées par P1, P2, P3 et P4 pouvant être utilisées, à des stades différentes, dans le procédé selon l'invention.
Pour les méthodes P1, P2 et P3 les paramètres numériques définissant les conditions expérimentales (paramètres opérationnels) sont donnés ici pour l'espèce *Gammarus fossarum.* Ils peuvent varier légèrement par rapport aux valeurs et fourchettes de valeurs préférées indiquées, à condition de vérifier que ces paramètres opérationnels représentent toujours le même phénomène biologique, et à condition de déterminer de nouveau les paramètres empiriques (paramètres résultants) indiqués. L'homme du métier sera en mesure de vérifier leur applicabilité, et le cas échéant déterminer leurs valeurs numériques pour les autres espèces du genre *Gammarus* et les autres familles de *Gammaridae.*

Méthode P1

Nous décrivons ici la méthode P1 permettant de prédire le pourcentage attendu de femelles en stade AB, C1, C2, D1 et D2 au bout de n jours lors d'une exposition à températures variables. Les paramètres chiffrés s'appliquent à l'espèce *Gammarus fossarum* ; pour d'autres espèces, d'autres genres et d'autres familles de crustacés ils doivent être déterminés au préalable.

1) À partir de l'enregistrement des températures effectué typiquement toutes les heures pendant les *n* jours, on calcule la température moyenne de chaque jour.
On dispose ainsi de *n* températures moyennes (en °C), une pour chaque jour : T1, T2, ..., Tn.

2) Pour chacune de ces températures moyennes, on calcule une durée d (en jours) qui correspond au temps qui serait nécessaire pour le gammare pour passer à cette température du début du cycle de mue (point de départ du test) au début du stade D2 (*i.e.* réalisation des 4 premiers stades). Pour cela on applique la formule empirique :

$$d = (31.92 - 0.5430 * T)/(-0.0125 + 0.0742 * T).$$

On obtient donc *n* durées théoriques de cycle : d1, d2, ..., dn.

3) Avec ces *n* durées, on calcule ensuite un pourcentage (*p*) de réalisation des 4 premiers stades du cycle à la fin des *n* jours. *p*= 1/d1 + 1/d2 +... + 1/dn.

4) A partir de ce pourcentage *p*, on calcule les probabilités d'avoir réalisé la transition entre chacun des 5 stades à la fin des *n* jours. Pour l'espèce *Gammarus fossarum* on utilise les formules empiriques suivantes :

a) Probabilité d'avoir réalisé la transition du stade B vers le stade C1 :

$$ABC1 = \exp(-3.7225 + 0.1101*p) / (1 + \exp(-3.7225 + 0.1101*p))$$

b) Probabilité d'avoir réalisé la transition du stade C1 vers le stade C2 :

$$C1C2 = \exp(-17.6788 + 0.2497*p) / (1 + \exp(-17.6788 + 02497*p))$$

c) Probabilité d'avoir réalisé la transition du stade C2 vers le stade D1 :

$$C2D1 = \exp(-59.3139 + 0.6655*p) / (1 + \exp(-59.3139 + 0.6655*p))$$

d) Probabilité d'avoir réalisé la transition du stade D1 vers le stade D2 :

$$D1D2 = \exp(-26.2050 + 0.2602*p) / (1 + \exp(-26.2050 + 0.2602*p))$$

5) A partir de ces 4 probabilités, on déduit les proportions de femelles attendues respectivement en stade AB, C1, C2, D1 et D2 au bout de *n* jours:

Proportion au stade AB = 1 - ABC1
Proportion au stade C1 = ABC1 - C1C2
Proportion au stade C2 = C1C2 - C2D1
Proportion au stade D1 = C2D1 - D1D2
Proportion au stade D2 = D1D2

Méthode P2 :

Nous décrivons ici une méthode pour déterminer la durée d'exposition du test de reprotoxicité *in situ.*

1) Après une première période d'exposition DE1 (préférentiellement 7 jours d'exposition), on récupère un premier enregistrement des températures effectué typiquement toutes les heures pendant la première période d'exposition.

2) A partir de cet enregistrement, on crée des enregistrements virtuels de températures prévisionnelles pour des durées d'exposition allant de 10 à 30 jours, en prolongeant les DE1 jours d'enregistrement par une duplication artificielle de la série des températures qui ont été enregistrées pendant les DE1 premiers jours.

3) On calcule, avantageusement à l'aide de l'algorithme indiqué ci-dessus (P1), la proportion de femelles attendue en stade C2, pour 10, 11, ..., 29, 30 jours d'exposition, en considérant les enregistrements de températures prévisionnelles.

4) On fixe la date d'arrêt de l'exposition *in situ* en choisissant la plus petite durée qui permet d'avoir au moins 80% de femelles en C2. On détermine ainsi une deuxième période d'exposition DE2.

5) À la récupération des systèmes d'exposition à la date ainsi choisie, l'enregistrement des températures réelles pendant le test est récupéré. A l'aide de la méthode P1 décrite ci-dessus, on vérifie si la proportion de femelles en stade C2 attendue est supérieure à 80%.

Si cette condition est remplie, on effectue les mesures biologiques (étape 5 (g)).

Si cette condition n'est pas remplie (*i.e.* si les températures d'exposition ont été plus faibles que dans le scénario prévisionnel), on maintient les gammares en stabulation pendant une durée post-exposition supplémentaire au laboratoire DPE (maximum 3 jours). Pour fixer la température de cette stabulation, on calcule à l'aide de l'algorithme P1 indiqué ci-dessus la proportion de femelles en C2 attendue si on ajoute DPE jours d'exposition à 10°C, 11°C, ..., 15°C, 16°C à l'enregistrement des températures. On choisit alors pour la stabulation la plus faible des températures qui permet d'obtenir au moins 80% des femelles en C2. Les mesures biologiques sont réalisées après cette stabulation.

Méthode P3 :

Nous décrivons ici en plus grand détail les trois critères dont la possible désynchronisation est utilisée dans la méthode selon l'invention comme un indicateur de la toxicité de l'eau à caractériser. Les paramètres chiffrés s'appliquent à l'espèce *Gammarus fossarum* ; pour d'autres espèces, d'autres genres et d'autres familles de crustacés ils doivent être déterminés au préalable.

(i) Critère du stade de mue : détermination du stade de mue

Ce critère se décline en cinq stades de mue $M_a$ où est un nombre entier de 1 à 5, soit $M_1$, $M_2$, $M_3$, $M_4$ et $M_5$.
Le stade $M_1$ est le stade de mue AB (regroupement des stades de post-mue A et B).
Le stade $M_2$ est le stade de mue C1.
Le stade $M_3$ est le stade de mue C2.
Le stade $M_4$ est le stade de mue D1.
Le stade $M_5$ est le stade de mue D2.

(ii) Critère de la vitellogénèse : détermination de la surface ovocytaire moyenne

Ce critère se décline en cinq stades de vitellogénèse $V_b$ où b est un nombre entier de 1 à 5, soit $V_1$, $V_2$, $V_3$, $V_4$ et $V_5$.
Le stade $V_1$ est le stade dans lequel la surface ovocytaire est comprise entre 20 100 $\mu m^2$ et 23 700 $\mu m^2$.
Le stade $V_2$ est le stade dans lequel la surface ovocytaire est comprise entre 34 900 $\mu m^2$ et 43 900 $\mu m^2$.
Le stade $V_3$ est le stade dans lequel la surface ovocytaire est comprise entre 99 200 $\mu m^2$ et 112 800 $\mu m^2$.
Le stade $V_4$ est le stade dans lequel la surface ovocytaire est comprise entre 129 700 $\mu m^2$ et 136 300 $\mu m^2$.
Le stade $V_5$ est le stade dans lequel la surface ovocytaire est comprise entre 158 200 $\mu m^2$ et 169 800 $\mu m^2$.
Pour une espèce de crustacés donnée, ces surfaces ovocytaires ne dépendent pas de la taille de l'individu, mais les indications chiffrées correspondent ici à l'espèce *Gammarus fossarum* ; pour d'autres espèces de crustacés ces valeurs doivent être déterminées au préalable.

(iii) Critère de l'embryogénèse : détermination du stade de développement embryonnaire

Ce critère se décline en cinq stades d'embryogénèse $E_c$ où c'est un nombre entier de 1 à 5, soit $E_1$, $E_2$, $E_3$, $E_4$ et $E_5$.

Le stade $E_1$ est le stade de l'embryon nouvellement fertilisé, ovale et indifférencié.

Le stade $E_2$ est le stade de l'apparition de la ventroflexion.

Le stade $E_3$ est le stade de l'apparition du céphalothorax et de la segmentation des appendices.

Le stade $E_4$ est le stade où les yeux sont clairement visibles et les appendices sont complètement développés.

Le stade $E_5$ est le stade du juvénile nouvellement éclos.

Méthode P4 : Critère de l'impact sur l'alimentation

Les paramètres chiffrés donnés pour cette méthode s'appliquent à l'espèce *Gammarus fossarum* ; pour d'autres espèces, d'autres genres et d'autres familles de crustacés ils doivent être déterminés au préalable. Des disques de feuilles sont préparés à partir de feuilles d'aulne séchées puis réhydratées dans de l'eau douce, renouvelée typiquement une fois par jour pendant deux jours (étape dite de « leaching »). Les feuilles sont ensuite étalées sur une planche puis découpées à l'emporte-pièce pour réaliser des disques de 2,2 centimètres de diamètre.

Dans le cas où il y a désynchronisation, on analyse s'il y a un impact sur l'alimentation. On détermine l'indice d'inhibition de l'alimentation (paramètre appelé « FI », Feeding Inhibition) qui exprime l'écart par rapport à la valeur attendue sans contamination pour la température du milieu d'exposition :

$$FI = (FR_{préd} - FR_i) / FR_{préd}) \times 100$$

où FI représente le taux d'inhibition en pourcents, $FR_{préd}$ exprimé en [mm$^2$ jour$^{-1}$ individu$^{-1}$] représente le taux d'alimentation attendu et $FR_i$ exprimé en [mm$^2$ jour$^{-1}$ individu$^{-1}$] représente le taux d'alimentation mesuré pour le réplicat i (voir ci-dessous).

Le taux d'alimentation prédit doit être corrigé en fonction de la température moyenne T de l'eau et de la taille moyenne des individus utilisés. Le taux d'alimentation prédit pour un réplicat d'individus d'une taille moyenne de 10,5 mm suit la relation linéaire suivante en fonction de la température :

$$FR_{préd} [mm^2 \ jour^{-1} \ individu^{-1}] = 1{,}85 \ T \ [°C] + 3{,}14$$

et doit être corrigé vers le haut ou vers le bas de 4,526 [mm$^2$ jour$^{-1}$ individu$^{-1}$] par mm de taille moyenne supplémentaire ou taille en moins ; cet ajustement en fonction de la taille est valable pour une gamme de tailles moyennes comprises entre 9.5 et 11.5 mm.

Le taux d'alimentation mesuré $FR_i$ est déterminé comme

$$FR_i = (2 \times S_{témoin} - S_i)/[(N + L_i)/2] \times 1/7$$

où $S_{témoin}$ représente la surface restante de 10 disques de feuilles témoins sans gammares, $S_i$ représente la surface restante des 20 disques de feuilles consommées pour le réplicat i, et $L_i$ représente le nombre de gammares vivants à l'arrêt de l'essai pour le réplicat i, et N représente le nombre de gammares au début de l'essai (typiquement 20).

Le test est considéré comme pertinent pour des survies supérieures à 75%. Le témoin comportant les disques de feuilles exposés sans gammares permet d'intégrer la dégradation des disques liée aux contraintes du milieu d'exposition. Le taux d'alimentation est exprimé en millimètres carré de surface consommée par jour et par gammare.

Nous donnons ici des précisions sur certaines des étapes du procédé complet de détermination du degré de contamination d'une eau douce afin de faciliter à l'homme du métier l'exécution du procédé selon l'invention.

Un premier aspect particulier de l'invention est que le procédé selon l'invention nécessite un contrôle de la température d'exposition des crustacés. La température de l'eau influe sur le métabolisme de *Gammarus fossarum,* et sur différents phénomènes et paramètres qui sont essentiels pour l'invention. La figure 3 montre l'influence de la température sur la consommation alimentaire. La figure 4 montre l'influence de la température du milieu aquatique sur la durée des différents stades du cycle de mue. Elle a été compilée à partir de mesures faites dans des conditions artificielles de température constante. On constate ainsi que pour les expérimentations en site réel, où l'expérimentateur ne peut pas agir sur la température du milieu aquatique pour la rendre constante, la durée d'exposition doit être adaptée à cette température ; le procédé selon l'invention permet cette adaptation en températures variables.

Précisions sur l'étape (1) : Les crustacés approvisionnés doivent être sains et doivent avoir vécu dans une eau propre. Ils peuvent être prélevés soit sur un site naturel dont la qualité de l'eau a été convenablement qualifiée au préalable, soit à partir d'un élevage en eau propre.

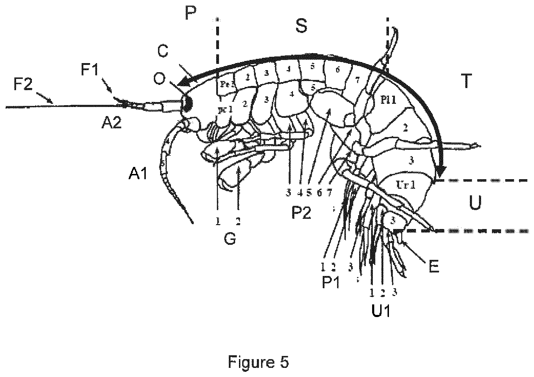

Précisions sur l'étape (3) : La figure 5 montre de manière schématique l'organisation générale d'un gammare.

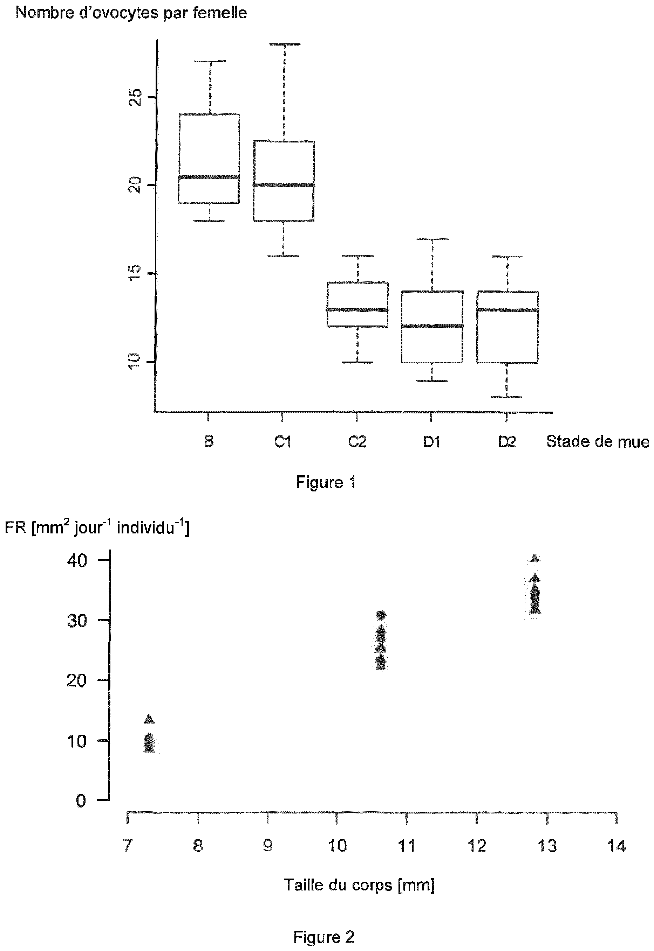

La ligne dorsale entre les deux flèches indique la mesure de sa taille. La figure 2 montre la relation entre la taille du corps et la consommation alimentaire moyenne de l'espèce *Gammarus fossarum.*

Précisions sur l'étape (2) : L'objectif de cette phase de stabulation (mise en quarantaine) est d'éliminer les individus affaiblis, d'assurer une homogénéité physiologique des organismes utilisés quelle que soit la saison (réserves énergétiques, taux d'activité comportementale), et de les acclimater aux futures conditions d'exposition.

Cette phase de stabulation peut se faire dans des aquariums contenant de préférence l'eau dans lequel les crustacés avaient été prélevés, renouvelée au goutte à goutte (typiquement le volume de l'aquarium est renouvelé quatre fois par jour) par une eau douce qui présente de préférence une conductivité proche de celle de l'eau à caractériser dans le futur site exposition (typiquement comprise entre 300 et 600 $\mu S$ cm$^{-1}$), et oxygénée en continu par bullage. Avantageusement la température est comprise entre 11°C et 13°C, et la photopériode est de 14 h jour / 10 h nuit. L'alimentation doit être adaptée à l'espèce de crustacé utilisée : pour l'espèce *Gammarus fossarum* on utilise avantageusement des feuilles d'aulne séchées (*Alnus glutinosa*) complétées par des vers du genre *Tubifex* une fois par semaine.

Précisions sur l'étape (3) : Cette étape est avantageusement effectuée en laboratoire quelques jours avant le début de l'exposition à l'eau à caractériser. Elle peut aussi être faite avant l'étape (2) (mise en quarantaine). Le tri peut s'effectuer sur une table lumineuse avec de l'eau du milieu de stabulation (en évitant réchauffement de l'eau par la source lumineuse et l'air ambiant). Pour la plupart des crustacés de la famille des *Gammaridae*, et notamment pour le genre *Gammarus* (et a *fortiori* pour l'espèce *Gammarus fossarum*), les femelles en reproduction sont identifiables à l'oeil nu par la présence d'ovocytes noirs dans le dos et/ou d'embryons dans le marsupium (poche incubatrice ventrale située sous l'abdomen), et leur état reproductif précis par rapport au stade de mue est également identifiable à l'oeil nu. Les mâles sont identifiables à l'oeil nu par l'absence d'ovocytes et d'embryons ; en cas de doute l'observation à la loupe binoculaire des papilles génitales permet de confirmer le phénotype mâle. On évite les individus parasités (présence de tâches orangées ou blanches, visibles notamment à travers la cuticule). Avantageusement on prévoit un nombre d'individus d'au moins 10% supérieur aux besoins pour pallier aux erreurs de tri et à la perte par la mortalité naturelle et le cannibalisme. Pour le tri des couples, on ne retient que les précopulats dont la femelle est en fin de phase de cycle reproductif. Pour cela, on sélectionne les femelles qui possèdent des ovaires très développés et des juvéniles au sein de leur marsupium.

Précisions sur l'étape (4) : Le transfert des individus des lots M et C du laboratoire vers le site d'essai (i.e. le cours d'eau à caractériser) est avantageusement effectué dans des conditions assurant une survie optimale des individus. Les individus sont mis en chambres d'encagement remplies d'une eau similaire à celle utilisée à l'étape 2, et qui présente une température et conductivité similaires à celle utilisées à l'étape 2. On prévoit une alimentation à volonté. On veillera à ce que la température de l'eau ne change pas de manière significative pendant le transfert. Les lots M et C sont avantageusement gardés séparés.

Pour un *Gammaridae* en bonne santé, qui n'a pas été altéré par des polluants, la consommation alimentaire augmente de manière linéaire avec la taille du corps, comme le montre la figure 2.

Précisions sur les étapes (5)(e) et 5(f) : Ces étapes sont optionnelles : après l'exposition *in situ* du lot C (étape 5 (c)), on détermine la température d'exposition pour ajuster le moment où l'on effectue les analyses biométriques typiquement au microscope (étape 5 (g)) et obtenir un maximum de femelles du lot C au stade de mue C2.

Précisions sur l'étape (5) (g)(i) : Chez les *Gammaridae*, et a *fortiori* dans *Gammarus fossarum,* le stade de développement embryonnaire peut être évalué sous un microscope optique (un grossissement d'environ 80x convient) après avoir retiré les embryons du marsupium et déposés sur une lame de verre. Voir aussi la description donnée en relation avec la méthode P3 pour la définition des stades de développement embryonnaire.

Précisions sur l'étape (5) (g)(ii) : Chez les *Gammaridae,* et a *fortiori* dans *Gammarus fossarum,* la surface ovocytaire (correspondant à la surface des ovocytes) est déterminée à partir d'observations des femelles (montées *in toto* entre deux lamelles de verre) au microscope optique (un grossissement d'environ 50x convient) ; ces observations peuvent être évaluées à l'aide d'un logiciel d'analyse d'images. Cela est décrit en détail dans l'article précité de Geffard et al., p. 2250.

Précisions sur l'étape (5) (g)(iii) : Chez les *Gammaridae,* et a *fortiori* dans *Gammarus fossarum,* le stade de mue est déterminé à partir de l'analyse fine des évolutions tégumentale du dactylopodite et du protopodite de la première et deuxième paire de périopodes ; cela est décrit en détail dans l'article précité de Geffard et al.

Précisions sur l'étape (6) : Chez les femelles saines et sexuellement actives de *Gammaridae*, et a *fortiori* de *Gammarus fossarum*, la vitellogenèse (maturation des gonades) et le développement des embryons dans le marsupium (i .e. l'embryogénèse) se déroulent de manière parfaitement synchrone, à chaque cycle de mue, à l'état naturel (i.e. en l'absence de polluants et autres éléments perturbateurs). Les juvéniles éclos, issus de la ponte précédente, sortent du marsupium peu de temps avant l'exuviation de leur mère. Suite à cette exuviation, la femelle pond des oeufs dans le marsupium qui seront aussitôt fécondés par un mâle. Parallèlement, dans les gonades, un nouveau lot d'ovocytes primaires entre en vitellogenèse. L'exuviation peut ainsi être considérée comme le point de départ et d'aboutissement de la maturation gonadique ainsi que du développement embryonnaire dans le marsupium. Chez les femelles saines et sexuellement actives, le cycle de mue s'effectue dans un temps précis, fonction de la température du milieu de vie.

Pour une espèce de *Gammaridae* donnée, la durée d'un cycle de mue dépend principalement de la température (voir la figure 4).

**Revendications**

**1.** Procédé pour déterminer l'état de reprotoxicité d'une eau douce en milieu naturel à l'aide de crustacés d'une espèce sélectionnée parmi les crustacés de la famille des *Gammaridae,* et de préférence sélectionnée parmi les crustacés du genre *Gammarus,* dans lequel procédé

(A) on approvisionne des crustacés d'une même espèce composés de mâles et de femelles en état de précopulation, à savoir :

- un premier lot M de mâles et
- un deuxième lot de crustacés adultes en couple C en état de précopulat, ledit lot C étant composé de mâles et de femelles, lesdites femelles étant sélectionnées de manière à être toutes prêtes à pondre et à muer dans le jour ou les deux jours qui suivent ;

(B) on met les lots M et C en cages dans l'eau douce à caractériser pendant une première période d'exposition de durée DE1 prédéterminée, et on détermine la température moyenne T1 de l'eau au cours de ladite première période d'exposition DE1 et on détermine la consommation d'aliments solides des crustacés du lot M au cours de ladite durée DE1 ;
(C) à l'issue de la durée d'exposition DE1 :

(a) on récupère le lot M, et
(b) on continue à exposer ledit lot C pendant une deuxième période d'exposition de durée DE2 prédéterminée en déterminant le pourcentage attendu de femelles en stade de mue AB, C1, C2, D1 et D2 au bout de n jours lors d'une exposition à températures variables, de la manière suivante :

(i) on détermine la température moyenne $T_n$ de chaque jour *n,*
(ii) pour chacune de ces *n* températures Tn on calcule une durée d en jours qui correspond au temps qui serait nécessaire pour le crustacé pour passer à cette température du début du cycle de mue initial au début du stade de mue D2,
(iii) en utilisant ces *n* durées d on calcule le pourcentage *p* de réalisation des 4 premiers stades de mue à la fin des *n* jours,
(iv) à partir du pourcentage *p* on calcule les probabilités *q* d'avoir réalisé la transition entre chacun des cinq stades à la fin des n jours,
(v) à partir de ces probabilités *q* on détermine les proportions de femelles attendues respectivement aux stades AB, C1, C2, D1 et D2 au bout de *n* jours.

(c) et on détermine la température moyenne T2 de l'eau au cours de cette deuxième période d'exposition DE2 ;
(d) on détermine pour chacun des crustacés femelles du lot C :

(i) le stade de développement embryonnaire (paramètre appelé ici « critère d'embryogénèse ») $E_c$, et
(ii) un paramètre $V_b$ qui représente la vitellogénèse, et qui est de manière préférée la surface ovocytaire (paramètre appelé ici « critère de vitellogénèse »), et
(iii) le stade de mue $M_a$;

(e) on évalue la synchronisation entre les critères du stade de mue, de vitellogénèse et d'embryogénèse, et

- si $M_a$, $V_b$ et $E_c$ sont synchronisés on évalue si la distribution statistique des femelles dans les différents stades de mue est conforme à une distribution de référence obtenue dans des conditions de non-toxicité.

**2.** Procédé selon la revendication 1, dans lequel si $M_a$, $V_b$ et $E_c$ sont synchronisés et la distribution statistique des femelles dans les différents stades de mue est conforme à une distribution de référence obtenue dans des conditions de non-toxicité, on conclut que l'eau à caractériser ne présente pas de toxicité, et si $M_a$, $V_b$ et $E_c$ sont synchronisés et la distribution statistique des femelles dans les différents stades de mue n'est pas conforme à une distribution de référence obtenue dans des conditions de non-toxicité, on conclut que l'eau à caractériser présente une toxicité générale.

3. Procédé selon la revendication 1, dans lequel si $M_a$, $V_b$ et $E_c$ ne sont pas synchronisés et on constate une inhibition de l'alimentation, on conclut que l'eau à caractériser présente une toxicité générale, et si $M_a$, $V_b$ et $E_c$ ne sont pas synchronisés et on constate l'absence d'inhibition de l'alimentation on conclut que l'eau à caractériser provoque une toxicité spécifique, de type perturbation endocrinienne,

4. Procédé selon l'une quelconque des revendications 1 à 3, dans lequel on approvisionne à l'étape (A) des crustacés de l'espèce *Gammarus fossarum* et à l'étape (C)(d) :

(a) pour déterminer le stade de mue $M_a$ on considère que

a = 1 pour le stade de mue AB qui regroupe les stades de post-mue A et B,
a = 2 pour le stade de mue C1,
a = 3 pour le stade de mue C2,
a = 4 pour le stade de mue D1,
a = 5 pour le stade de mue D2,

(b) pour déterminer le critère de la vitellogénèse $V_b$ on considère que :

b = 1 si la surface ovocytaire est comprise entre 20 100 $\mu m^2$ et 23 700 $\mu m^2$,
b = 2 si la surface ovocytaire est comprise entre 34 900 $\mu m^2$ et 43 900 $\mu m^2$,
b = 3 si la surface ovocytaire est comprise entre 99 200 $\mu m^2$ et 112 800 $\mu m^2$,
b = 4 si la surface ovocytaire est comprise entre 129 700 $\mu m^2$ et 136 300 $\mu m^2$,
b = 5 si la surface ovocytaire est comprise entre 158 200 $\mu m^2$ et 169 800 $\mu m^2$ ;

(c) pour déterminer le critère de l'embryogénèse $E_c$ on considère que :

c = 1 au stade de l'embryon nouvellement fertilisé, ovale et indifférencié,
c = 2 au stade de l'apparition de la ventroflexion,
c = 3 au stade de l'apparition du céphalothorax et de la segmentation des appendices,
c = 4 au stade où les yeux sont clairement visibles (ommatidies) et les appendices sont complètement développés,
c = 5 au stade du juvénile nouvellement éclos.

5. Procédé selon la revendication 4, dans lequel on considère que $M_a$, $V_b$ et $E_c$ sont synchronisés si a = b = c, et que $M_a$, $V_b$ et $E_c$ ne sont pas synchronisés si cette condition a = b = c n'est pas remplie.

6. Procédé selon l'une quelconque des revendications 1 à 5, dans lequel on détermine l'inhibition de l'alimentation en alimentant les crustacés avec une source d'alimentation solide en feuilles de surface prédéterminée.

7. Procédé selon l'une quelconque des revendications 1 à 6, dans lequel on détermine la durée DE2 de ladite deuxième période d'exposition à partir de la température moyenne T1 de l'eau au cours de ladite première durée d'exposition de durée DE1.

**Patentansprüche**

1. Verfahren zum Bestimmen des Reprotoxizitätszustands eines Süßwassers in natürlicher Umgebung mithilfe von Krebstieren einer Spezies ausgewählt aus den Krebstieren der Familie der *Gammaridae,* und vorzugsweise ausgewählt aus den Krebstieren der Gattung *Gammarus,* wobei bei dem Verfahren

(A) Krebstiere ein und derselben Spezies beschafft werden, bestehend aus Männchen und Weibchen in Präkopulationszustand, nämlich:

- ein erstes Los M Männchen, und
- ein zweites Los erwachsener Krebstiere in Paarung C in Präkopulationszustand, wobei das Los C aus Männchen und Weibchen besteht, wobei die Weibchen so ausgewählt werden, dass sie alle bereit sind, am Tag oder den zwei Tagen, die folgen, zu legen und sich zu häuten;

(B) die Lose M und C in Käfigen für einen ersten Expositionszeitraum von vorbestimmter Dauer DE1 in das Süßwasser, das charakterisiert werden soll, gebracht werden und die mittlere Temperatur T1 des Wassers im Lauf des ersten Expositionszeitraums DE1 bestimmt wird, und der Verzehr an festen Nahrungsmitteln der Krebstiere des Loses M im Lauf der Dauer DE1 bestimmt wird;
(C) am Ende der Expositionsdauer DE1:

(a) das Los M eingesammelt wird, und
(b) das Los C für einen zweiten Expositionszeitraum von vorbestimmter Dauer DE2 weiter exponiert wird, unter Bestimmung des erwarteten Prozentsatzes an Weibchen in Häutungsstadium AB, C1, C2, D1 und D2 nach Ablauf von n Tagen bei einer Exposition mit variablen Temperaturen in der folgenden Weise:

(i) es wird die mittlere Temperatur $T_n$ jedes Tages $n$ bestimmt,
(ii) für jede dieser $n$ Temperaturen $T_n$ wird eine Dauer $d$ in Tagen berechnet, die der Zeit entspricht, welche für das Krebstier erforderlich wäre, um bei dieser Temperatur vom Beginn des anfänglichen Häutungszyklus zum Beginn des Häutungsstadiums D2 zu gelangen,
(iii) unter Verwendung dieser $n$ Dauern $d$ wird der Prozentsatz $p$ des Vollzugs der 4 ersten Häutungsstadien am Ende der $n$ Tage berechnet,
(iv) ausgehend vom Prozentsatz $p$ werden die Wahrscheinlichkeiten $q$, am Ende der n Tage den Übergang zwischen jedem der fünf Stadien vollzogen zu haben, berechnet,
(v) ausgehend von diesen Wahrscheinlichkeiten $q$ werden die erwarteten Anteile von Weibchen nach Ablauf von n Tagen jeweils in den Stadien AB, C1, C2, D1 und D2 berechnet,

(c) und es wird die mittlere Temperatur T2 des Wassers im Lauf dieses zweiten Expositionszeitraums DE2 berechnet;
(d) für jedes der weiblichen Krebstiere des Loses C werden bestimmt:

(i) das embryonale Entwicklungsstadium (Parameter, der hier "Embryogenesekriterium" genannt wird) $E_c$, und
(ii) ein Parameter $V_b$, der die Vitellogenese wiederspiegelt und der vorzugsweise die Ovozytenoberfläche ist (Parameter, der hier "Vitellogenesekriterium" genannt wird), und
(iii) das Häutungsstadium $M_a$;

(e) die Synchronisierung zwischen den Kriterien des Häutungs-, des Vitellogenese- und des Embryogenesestadiums evaluiert wird, und - wenn $M_a$, $V_b$ und $E_c$ synchronisiert sind, evaluiert wird, ob die statistische Verteilung der Weibchen in den verschiedenen Häutungsstadien mit einer Referenzverteilung übereinstimmt, die unter Bedingungen der Nicht-Toxizität erhalten wird.

2. Verfahren nach Anspruch 1, wobei wenn $M_a$, $V_b$ und $E_c$ synchronisiert sind und die statistische Verteilung der Weibchen in den verschiedenen Häutungsstadien mit einer Referenzverteilung übereinstimmt, die unter Bedingungen der Nicht-Toxizität erhalten wird, schlussgefolgert wird, dass das Wasser, welches charakterisiert werden soll, keine Toxizität aufweist, und wenn $M_a$, $V_b$ und $E_c$ synchronisiert sind und die statistische Verteilung der Weibchen in den verschiedenen Häutungsstadien nicht mit einer Referenzverteilung übereinstimmt, die unter Bedingungen der Nicht-Toxizität erhalten wird, schlussgefolgert wird, dass das Wasser, welches charakterisiert werden soll, eine allgemeine Toxizität aufweist.

3. Verfahren nach Anspruch 1, wobei wenn $M_a$, $V_b$ und $E_c$ nicht synchronisiert sind und eine Hemmung der Nahrungsaufnahme festgestellt wird, schlussgefolgert wird, dass das Wasser, welches charakterisiert werden soll, eine allgemeine Toxizität aufweist, und wenn $M_a$, $V_b$ und $E_c$ nicht synchronisiert sind und das Fehlen einer Hemmung der Nahrungsaufnahme festgestellt wird, schlussgefolgert wird, dass das Wasser, welches charakterisiert werden soll, eine spezifische Toxizität vom Typ endokrine Störung bewirkt.

4. Verfahren nach einem der Ansprüche 1 bis 3, wobei im Schritt (A) Krebstiere der Spezies *Gammarus fossarum* beschafft werden, und im Schritt (C)(d):

(a) um das Häutungsstadium $M_a$ zu bestimmen davon ausgegangen wird, dass

a = 1 für das Häutungsstadium AB, welches die Post-Häutungsstadien A und B zusammenfasst,
a = 2 für das Häutungsstadium C1,
a = 3 für das Häutungsstadium C2,
a = 4 für das Häutungsstadium D1,
a = 5 für das Häutungsstadium D2,
(b) um das Kriterium der Vitellogenese $V_b$ zu bestimmen davon ausgegangen wird, dass:

b = 1, wenn die Ovozytenoberfläche im Bereich zwischen 20 100 $\mu m^2$ und 23 700 $\mu m^2$ beträgt,
b = 2, wenn die Ovozytenoberfläche im Bereich zwischen 34 900 $\mu m^2$ und 43 900 $\mu m^2$ beträgt,
b = 3, wenn die Ovozytenoberfläche im Bereich zwischen 99 200 $\mu m^2$ und 112 800 $\mu m^2$ beträgt,
b = 4, wenn die Ovozytenoberfläche im Bereich zwischen 129 700 $\mu m^2$ und 136 300 $\mu m^2$ beträgt,
b = 5, wenn die Ovozytenoberfläche im Bereich zwischen 158 200 $\mu m^2$ und 169 800 $\mu m^2$ beträgt;

(c) um das Kriterium der Embryogenese $E_c$ zu bestimmen davon ausgegangen wird, dass:

c = 1 im Stadium des frisch befruchteten, ovalen und undifferenzierten Embryos,
c = 2 im Stadium des Auftauchens der Ventroflexion,
c = 3 im Stadium des Auftauchens des Cephalothorax und der Segmentation der Glieder,
c = 4 im Stadium, in dem die Augen deutlich sichtbar sind (Ommatidia) und die Glieder vollständig entwickelt sind,
c = 5 im Stadium des frisch geschlüpften Jungtiers.

5. Verfahren nach Anspruch 4, wobei davon ausgegangen wird, dass $M_a$, Vb und $E_c$ synchronisiert sind, wenn a = b = c, und dass $M_a$, $V_b$ und $E_c$ nicht synchronisiert sind, wenn diese Bedingung a = b = c nicht erfüllt ist.

6. Verfahren nach einem der Ansprüche 1 bis 5, wobei die Hemmung der Nahrungsaufnahme unter Ernähren der Krebstiere mit einer festen Nahrungsquelle aus Blättern von vorbestimmter Oberfläche bestimmt wird.

7. Verfahren nach einem der Ansprüche 1 bis 6, wobei die Dauer DE2 des zweiten Expositionszeitraums ausgehend von der mittleren Temperatur T1 des Wassers im Lauf des ersten Expositionszeitraums DE1 bestimmt wird.

**Claims**

1. Method for determining the state of reprotoxicity of freshwater in a natural environment using crustaceans of a species selected from crustaceans of the family of *Gammaridae,* and preferably selected from crustaceans of the genus *Gammarus,* in which method

(A) crustaceans of the same species comprised of males and females in a state of precopulation are supplied, namely;

- a first batch M of males and
- a second batch of adult crustaceans in pairs C in a precopulatory state, said batch C being comprised of males and females, said females being selected in such a way as to all be ready to spawn and moult within the following day or two days;

(B) batches M and C are placed in cages in freshwater to be characterised during a first period of exposure of a predetermined duration DE1, and the average temperature T1 of the water over the course of said first period of exposure DE1 is determined and the solid food consumption of the crustaceans of batch M during said duration DE1 is determined;
(C) at the end of the exposure duration DE1:

(a) the batch M is recovered, and
(b) said lot C continues to be exposed for a second period of exposure of a predetermined duration DE2 by determining the expected percentage of females in moulting stage AB, C1, C2, D1 and D2 at the end of n days during exposure to variable temperatures, in the following way:

(i) the average temperature $T_n$ of each day n is determined,

(ii) for each one of these n temperatures Tn a duration d in days is calculated which corresponds to the time that would be required for the crustacean to pass at this temperature from the beginning of the initial moulting cycle to the beginning of moulting state D2,

(iii) these *n* durations *d* are used to calculate the percentage *p* of the realisation of the first 4 stages of moulting at the end of the n days,

(iv) using the percentage *p* the probabilities *q* of having realised the transition between each one of the five stages at the end of the n days are calculated,

(v) using these probabilities *q* the proportions of expected females respectively at stages AB, C1, C2, D1 and D2 at the end of n days are determined.

(c) the average temperature T2 of the water during this second period of exposure DE2 is determined;

(d) the following is determined for each one of the female crustaceans of batch C:

(i) the stage of embryonic development (parameter here called "embryogenesis criterion") $E_c$, and

(ii) a parameter $V_b$ which represents vitellogenesis, and which is preferably the ovocyte surface (parameter here called "vitellogenesis criterion"), and

(iii) the moulting stage $M_a$:

(e) the synchronisation is evaluated between the criteria of the moulting stage, vitellogenesis and embryogenesis, and

- if $M_a$, $V_b$ and $E_c$ are synchronised it is evaluated whether the statistical distribution of the females in the various stages of moulting is compliant with a reference distribution obtained in conditions of non-toxicity.

2. Method according to claim 1, wherein if $M_a$, $V_b$ and $E_c$ are synchronised and the statistical distribution of the females in the various stages of moulting is compliant with a reference distribution obtained in conditions of non-toxicity, the conclusion is made that the water to be characterised does not have any toxicity, and if $M_a$, $V_b$ and $E_c$ are synchronised and the statistical distribution of the females in the various stages of moulting is not compliant with a reference distribution obtained in conditions of non-toxicity, the conclusion is made that the water to be characterised has a general toxicity.

3. Method according to claim 1, wherein if $M_a$, $V_b$ and $E_c$ are not synchronised and feeding inhibition is observed, the conclusion is made that the water to be characterised has a general toxicity, and if $M_a$, $V_b$ and $E_c$ are not synchronised and the absence of feeding inhibition is observed the conclusion is made that the water to be characterised is causing a specific toxicity, of the endocrine disruption type.

4. Method according to any of claims 1 to 3, in which in the step (A) crustaceans of the species *Gammarus fossarum* are supplied and in the step (C)(d):

(a) in order to determine the moulting stage $M_a$ it is considered that
a = 1 for the moulting stage AB that groups together the post-moulting stages A and B,
a = 2 for the moulting stage C1,
a = 3 for the moulting stage C2,
a = 4 for the moulting stage D1,
a = 5 for the moulting stage D2,
(b) in order to determine the vitellogenesis criterion $V_b$ it is considered that;
b = 1 if the ovocyte surface is between 20,100 $\mu m^2$ and 23,700 $\mu m^2$,
b = 2 if the ovocyte surface is between 34,900 $\mu m^2$ and 43,900 $\mu m^2$,
b = 3 if the ovocyte surface is between 99,200 $\mu m^2$ and 112,800 $\mu m^2$,
b = 4 if the ovocyte surface is between 129,700 $\mu m^2$ and 136,300 $\mu m^2$,
b = 5 if the ovocyte surface is between 158,200 $\mu m^2$ and 169,800 $\mu m^2$ ;
(c) in order to determine the embryogenesis criterion $E_c$ it is considered that;
c = 1 at the stage of the newly fertilised embryo, oval and undifferentiated,
c = 2 at the stage of the appearance of ventroflexion,
c = 3 at the stage of the appearance of the cephalothorax and of the segmentation of the appendages,
c = 4 at the stage when the eyes are clearly visible (ommatidia) and the appendages are fully developed,
c = 5 at the newly hatches juvenile stage.

5.  Method according to claim 4, wherein it is considered that $M_a$, $V_b$ and $E_c$ are synchronised if a = b = c, and that $M_a$, $V_b$ and $E_c$ are not synchronised if this condition a = b = c is not satisfied.

6.  Method according to any of claims 1 to 5, wherein the feeding inhibition is determined by supplying the crustaceans with a source of solid food in sheets of a predetermined surface.

7.  Method according to any of claims 1 to 6, wherein the duration DE2 of said second period of exposure is determined from the average temperature T1 of the water during said first period of exposure of duration DE1.

Nombre d'ovocytes par femelle

Figure 1

FR [mm² jour⁻¹ individu⁻¹]

Figure 2

Figure 3

Figure 4

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2013081496 A
- RU 2491546
- RU 2377560
- DE 4110877
- WO 90112886 A
- DE 19848230, Almut Gerhardt
- DE 4313109, Liess
- SU 2029948
- WO 9013028 A

- WO 9002947 A
- EP 2177905 A
- WO 2008150096 A
- EP 1256800 A
- DE 4443788
- US 2004147030 A
- US 6365129 B
- US 5798222 A
- RU 2057337


**Littérature non-brevet citée dans la description**

- **B. XUEREB et al.** Acetylcholinesterase activity in Gammarus fossarum (Crustacea Amphpoda) - Intrinsic variability, reference levels, and a reliable tool for field surveys. *Aquatic Toxicology,* 2009, vol. 93, 225-233
- **E. LACAZE et al.** Linking genotoxic responses in Gammarus fossarum germ cells with reproduction impairement, using the Comet assay. *Environmental Research,* 2011, vol. 111, 626-634
- **G. JUBEAUX et al.** Vitellogenin-like proteins in the freshwater amphipod Gammarus fossarum (Koch, 1835): Functional characterization throughout reproductive process, potential or use as an indicator of oocyte quality and endocrine disruption biomarkers in males. *Aquatic Toxicology,* 2012, vol. 112-113, 72-82

- **O. GEFFARD et al.** Ovarian cycle and embryonic development in Gammarus fossarum: Application for reproductive toxicity assessment. *Environmental Toxicology and Chemistry,* 2010, vol. 29 (10), 2249-2259
- **R. COULAUD et al.** In situ feeding assay with Gammarus fossarum (Crustacea): Modelling the influence of confounding factors to improve water quality biomonitoring. *Water Research,* 2011, vol. 45, 6417-6429